(19) Europäisches Patentamt / European Patent Office / Office européen des brevets

(11) **EP 1 925 292 A1**

(12) **DEMANDE DE BREVET EUROPEEN**

(43) Date de publication:
  **28.05.2008 Bulletin 2008/22**

(51) Int Cl.:
  *A61K 8/37* *(2006.01)*   *A61Q 1/06* *(2006.01)*
  *A61Q 19/00* *(2006.01)*

(21) Numéro de dépôt: **07121283.1**

(22) Date de dépôt: **22.11.2007**

(84) Etats contractants désignés:
  **AT BE BG CH CY CZ DE DK EE ES FI FR GB GR HU IE IS IT LI LT LU LV MC MT NL PL PT RO SE SI SK TR**
  Etats d'extension désignés:
  **AL BA HR MK RS**

(30) Priorité: **23.11.2006  FR 0655074**
        **23.11.2006  FR 0655063**
        **23.11.2006  FR 0655069**
        **23.11.2006  FR 0655071**

(71) Demandeur: **L'Oréal**
  **75008 Paris (FR)**

(72) Inventeurs:
  • **Fouron, Jean-Yves**
    **92340, Bourg la Reine (FR)**
  • **Auguste, Frédéric**
    **94550, Chevilly-Larue (FR)**

(74) Mandataire: **Duvert, Sandra**
  **L'Oréal**
  **DIPI**
  **River Plaza**
  **25-29 Quai Aulagnier**
  **92665 Asnières-sur-Seine (FR)**

(54) **Composition cosmétique comprenant au moins un ester d'acide carbonique volatil**

(57)  La présente demande concerne une composition cosmétique comprenant au moins un ester d'acide carbonique répondant à la formule (1) suivante :

$$R_1-O-CO-O-R_2 \qquad (I)$$

dans laquelle R1 et R2 sont identiques ou différents et représentent un radical hydrocarboné linéaire ou ramifié, avec le proviso que
- lorsque R1 et R2 sont linéaires, ils contiennent chacun de 1 à 6 atomes de carbone avec $6 \leq R_1 + R_2 \leq 7$ ; et
- lorsque R1 et/ou R2 sont ramifiés, ils contiennent chacun de 1 à 8 atomes de carbone avec $6 \leq R_1 + R_2 \leq 9$.
  Cette composition peut être utilisée en tant que produit de soin et/ou de maquillage des matières kératiniques, notamment de la peau, des lèvres et/ou des phanères.

EP 1 925 292 A1

**Description**

La présente invention se rapporte à une composition cosmétique, notamment une composition cosmétique de maquillage ou de soin de la peau du visage ou du corps humain, du cuir chevelu, des lèvres ou des phanères, comme les cheveux, les cils, les sourcils ou les ongles.

La composition de l'invention peut en particulier constituer un produit de soin, de coiffage ou de maquillage des lèvres, du corps ou des phanères pouvant être doté en outre de propriétés de soin. La composition de l'invention peut constituer notamment un rouge à lèvres ou un brillant à lèvres, un fard à joues ou à paupières, un produit pour tatouage, un mascara, un eye-liner, un produit de bronzage artificiel de la peau, un fond de teint ou une crème de soin.

Les compositions cosmétiques doivent généralement présenter certaines propriétés telles que la tenue, la non-migration, le non-transfert, le play-time, le glissant à l'application (ou bon étalement), le confort, la brillance ou encore la couvrance. Une même composition ne doit pas nécessairement présenter toutes ces propriétés, cependant, dans la majorité des cas, on chercher à ce que la composition possède au moins quelques unes d'entre elles.

La tenue de la composition pourra en particulier être la tenue à l'eau ou aux frottements des doigts ou bien encore aux larmes, à la sueur ou au sébum.

La propriété de non-migration correspond pour une composition à ne pas migrer dans les plis de la peau comme les rides ou ridules situées autour des lèvres et des yeux (paupières notamment).

Le caractère non-transfert d'une composition correspond au fait qu'une fois appliquée, celle-ci ne se dépose pas de façon notable sur les surfaces avec lesquelles elles viennent en contact (verre, tasse, cigarette, vêtement par exemple).

Le play-time d'un produit correspond au temps pendant lequel le consommateur peut travailler celui-ci lors de son application et traduit donc la facilité d'application du produit.

Les compositions cosmétiques comprennent fréquemment une phase grasse contenant un solvant volatil. En effet, celui-ci permet d'apporter une évolution des propriétés du produit pendant et après le dépôt, conduisant alors, suivant le produit cosmétique envisagé, à des propriétés de tenue du produit déposé, de confort ou de texture lors de l'application du produit, ainsi qu'à des propriétés mécaniques ou optiques spécifiques des dépôts.

Ces solvants volatils sont classiquement utilisés dans les domaines du soin, de l'hygiène, des produits capillaires, des parfums et du maquillage, dans des galéniques très variées : émulsions directes ou inverses, pâtes anhydres, sticks anhydres, émulsions solides, ...

Dans le cadre de la formulation de compositions cosmétiques, il serait avantageux de disposer de nouveaux solvants volatils, permettant l'obtention de compositions présentant au moins certaines des propriétés énoncées ci-avant.

La Demanderesse a découvert de manière inattendue qu'une catégorie particulière de composés répond aux critères énoncés ci-avant et confèrent ainsi aux compositions d'excellentes propriétés cosmétiques telles que le bon étalement et/ou le toucher non gras et/ou le confort et/ou le non-transfert et/ou la non-migration par exemple. Cette liste est non exhaustive et plus généralement, ces composés présentent une bonne compatibilité avec d'autres constituants usuellement présents dans les compositions cosmétiques et confèrent à la composition la plupart des propriétés classiquement souhaitées.

De façon plus précise, l'invention a pour objet une composition cosmétique comprenant, dans un milieu physiologiquement acceptable, au moins un ester d'acide carbonique répondant à la formule (I) suivante :

$$R_1\text{-}O\text{-}CO\text{-}O\text{-}R_2 \qquad (I)$$

dans laquelle R1 et R2 sont identiques ou différents et représentent un radical hydrocarboné linéaire ou ramifié, avec le proviso que

- lorsque R1 et R2 sont linéaires, ils contiennent chacun de 1 à 6 atomes de carbone avec $6 \leq R_1 + R_2 \leq 7$ ; et
- lorsque R1 et/ou R2 sont ramifiés, ils contiennent chacun de 1 à 8 atomes de carbone avec $6 \leq R_1 + R_2 \leq 9$.

L'expression « $6 \leq R_1 + R_2 \leq 7$ » signifie que la somme des atomes de carbone de R1 et R2 est comprise entre 6 et 7. De la même manière, « $6 \leq R_1 + R_2 \leq 9$ » signifie que la somme des atomes de carbone de R1 et R2 est comprise entre 6 et 9.

Par "hydrocarboné", on entend un radical ou un composé formé essentiellement, voire constitué, d'atomes de carbone et d'hydrogène, et éventuellement d'atomes d'oxygène, d'azote, de soufre, de phosphore, et ne contenant pas d'atome de silicium ou de fluor. Elle peut contenir des groupes alcool, éther, acide carboxylique, amine et/ou amide. De préférence, le l'adjectif « hydrocarboné » désigne un radical ou un composé constitué uniquement d'atomes de carbone et d'hydrogène, tel que les radicaux alkyl, alkényl ou alkynyl par exemple.

Dans la formule (I) précédemment citée, R1 et R2 sont choisis indépendamment l'un de l'autre et sont deux radicaux distincts, c'est-à-dire qu'ils ne sont pas liés l'un à l'autre par une liaison covalente.

De façon préférée, la composition selon l'invention est une composition cosmétique de maquillage ou de soin de matières kératiniques.

Dans le cadre de la présente invention, « matières kératiniques » comprend la peau, les lèvres, les ongles, les cheveux, les cils et les sourcils et « fibres kératiniques » les cheveux , les cils et les sourcils.

L'expression « l'ester carbonique de formule (I) » ou « l'ester carbonique» recouvre le cas où il s'agit d'un ou plusieurs composés individuels ainsi que leur mélange. Ainsi, cette expression couvre « au moins un ester carbonique de formule (I)».

De façon préférée, les radicaux hydrocarbonés R1 et R2 sont aliphatiques.

De façon avantageuse, R1 et R2 sont des radicaux alkyl.

Selon un premier mode de réalisation de l'invention, R1 et R2 sont linéaires. Parmi ces esters d'acide carbonique, on peut citer :

Les esters d'acide carbonique linéaires comprenant 7 atomes de carbone tels que :

- Carbonate de méthyle et de n-pentyle ;

- Carbonate de n-butyle et d'éthyle;

- Carbonate de dipropyle.

Les esters d'acide carbonique linéaires comprenant 8 atomes de carbone tels que :

- Carbonate d'éthyle et de n-pentyle ;

- Carbonate de n-butyle et de n-propyle ;

- Carbonate de n-hexyle et de méthyle.

Selon un second mode de réalisation de l'invention, R1 et/ou R2 sont ramifiés. Par structure ramifiée, il faut comprendre que les esters d'acide carbonique possèdent au moins une ramification, de longueur minimale correspondant à un groupe méthyle -$CH_3$. En d'autres termes, sont considérés comme ramifiés les esters d'acide carbonique possédant au total au moins 3 groupes -$CH_3$ sur l'ensemble de la molécule.

On prévoit ainsi selon différentes variantes de l'invention que seul R1 ou seul R2 est ramifié, ou bien que R1 et R2 soient tous deux ramifiés.

De façon avantageuse, les esters d'acide carbonique de formule (I) ne comprennent pas plus de 4 ramifications, ou mieux pas plus de 2 ou 3 ramifications.

De façon encore préférée, les esters d'acide carbonique de formule (I) ne comprennent pas plus d'une ramification.

Le nombre de ramifications correspond au nombre de groupes -CH3 sur l'ensemble de la molécule auquel on retranche deux. Ainsi, par exemple l'acide carbonique, 3,3-diméthylbutyl méthyl ester présente quatre groupes -CH3 sur l'ensemble de la molécule et compte donc deux ramifications. On notera que le nombre de ramifications ne correspond donc pas forcément au nombre de carbones ramifiés. Dans la molécule citée précédemment en exemple, il n'y a en effet qu'un seul carbone ramifié et deux ramifications. D'une façon plus générale, le nombre de ramifications d'une molécule correspond au nombre de groupes latéraux contenant au moins un atome de carbone et branchés sur la chaîne principale de la molécule, la chaîne principale correspondant à la plus longue chaîne carbonée de la molécule (voir Organic Chemistry, S.H. Pine, 5ème Edition ; Mc Graw-Hill, chapitre 3).

De façon préférée, R1 et/ou R2 ne comprend pas de carbone quaternaire. Par carbone quaternaire, on entend un carbone ne portant pas d'atome d'hydrogène et lié à quatre autres atomes de carbone.

Parmi les esters d'acide carbonique ramifiés à 7 atomes de carbone, on peut citer :

Carbonate de 1-méthyléthyle et de n-propyle ;
Carbonate de 2,2-diméthylpropyle et de méthyle ;
Carbonate de méthyle et de 3-méthylbutyle ;
Carbonate de méthyle et de 2-méthylbutyle ;
Carbonate de méthyle et de 1-méthylbutyle ;
Carbonate de 1-éthylpropyle et de méthyle ;
Carbonate de 1,1-diméthyléthyle et d'éthyle ;
Carbonate d'éthyle et de 2-méthylpropyle ;

Carbonate de 1,1-diméthylpropyle et de méthyle ;
Carbonate de bis(1-méthyléthyl).

Parmi les esters d'acide carbonique ramifiés à 8 atomes de carbone, on peut citer :

Carbonate de 3,3-diméthylbutyle et de méthyle ;
Carbonate de 1-éthyl-1-méthylpropyle et de méthyle ;
Carbonate de butyle et de 1-méthyléthyle ;
Carbonate de 1-méthylpropyle et de propyle ;
Carbonate de 1,1-diméthyléthyle et de propyle ;
Carbonate de 2-éthylbutyle et de méthyle ;
Carbonate d'éthyle et de 1-éthylpropyle ;
Carbonate de 1-méthyléthyle et de 1-méthylpropyle ;
Carbonate de 1,1-diméthyléthyle et de 1-méthyléthyle ;
Carbonate de méthyle et de 1-méthylpentyle ;
Carbonate de 2-méthylpropyle et de propyle ester ;
Carbonate de méthyle et de 1,1,2-triméthylpropyle ;
Carbonate de 1,3-diméthylbutyle et de méthyle ;
Carbonate de 1,1-diméthylpropyle et d'éthyle.

Parmi les esters d'acide carbonique ramifiés à 9 atomes de carbone, on peut citer :

Carbonate de butyle et de 1-méthylpropyle ;
Carbonate de butyle et de 2-méthylpropyle ;
Carbonate d'isoheptyle et de méthyle ;
Carbonate de bis(1,1-diméthyléthyle) ;
Carbonate de 1,1-diéthylpropyle et de méthyle ;
Carbonate de bis(2-méthylpropyle) ;
Carbonate de bis(1-méthylpropyl) ;
Carbonate de butyle et de 1-méthylpropyle ;
Carbonate de butyle et de 1,1-diméthyléthyle ;
Carbonate d'éthyle et de 1-méthylpentyle.

Parmi les esters d'acide carbonique ramifiés à 10 atomes de carbone, on peut citer :

Carbonate de 1-méthylpentyle et de propyle ;

Carbonate de 2-méthylpentyle et de propyle ;

Carbonate de 1,2-diméthyl-1-(1-méthyléthyl)propyle et de méthyle ;

Carbonate de méthyle et de 1-méthylheptyle ;

Carbonate de méthyle et de 1-méthylheptyle ;

Carbonate de 2-méthylpropyle et de pentyle ;

Carbonate de 1,1-diméthylbutyle et de 1-méthyléthyle ;

Carbonate de 1,1-diméthyléthyle et de 1,1-dimethylpropyle ;

Carbonate d'isooctyle et de méthyle ;

Carbonate de 2-éthylhexyle et de méthyle.

De façon préférée, les esters d'acide carbonique selon l'invention ont des vitesses d'évaporation telles que la quantité évaporée en 30 minutes est comprise entre 4.1 mg/cm$^2$ et 24 mg/cm$^2$ (lorsqu'elle est mesurée sur le composé seul, dans les conditions définies ci-après).

La mesure de la volatilité d'un solvant est décrite dans la demande de brevet WO 06/013413, en fonction de la quantité évaporée en 30 minutes, selon le protocole défini ci-après :

Mesure de la vitesse d'évaporation d'un solvant (protocole)

On introduit dans un cristallisoir (diamètre : 7 cm) placée sur une balance se trouvant dans une enceinte d'environ 0,3 m3 régulée en température (25 °C) et en hygrométrie (humidité relative 50 %) 15 g d'huile ou du mélange d'huiles à tester. On laisse le liquide s'évaporer librement, sans l'agiter, en assurant une ventilation par un ventilateur (PAPST -MOTOREN, référence 8550 N, tournant à 2700 tours/minute) disposé en position verticale au-dessus du cristallisoir contenant le solvant, les pales étant dirigées vers le cristallisoir et à une distance de 20 cm par rapport au fond du cristallisoir. On mesure à intervalles réguliers la masse d'huile restant dans le cristallisoir. Les vitesses d'évaporation sont exprimées en mg d'huile évaporée par unité de surface (cm2) et par unité de temps (minute).

A titre indicatif la quantité en masse d'huile volatile évaporée au bout de 30 minutes pour certaines huiles volatiles selon ce protocole est donnée ci-après :

- isododécane : 24 mg/cm2,
- octaméthyl cyclotétrasiloxane (D4) : 18,7 mg/cm2,
- décaméthyl cyclohexasiloxane (D5) : 4,1 mg/cm2.

De façon préférée, les esters d'acide carbonique selon l'invention présentent un point éclair compris entre 43 et 100°C, et plus particulièrement entre 45 et 80°C. A titre indicatif, les points éclairs de l'isododécane et de la cyclométhicone D5 sont respectivement 45°C et 77°C.

L'ester d'acide carbonique de formule (I) peut être utilisé comme seul solvant lipophile volatil ou en mélange avec d'autres solvants (également appelés « huiles ») volatils lipophiles additionnels ne répondant pas à la formule (I).

Dans le cas d'un mélange de solvants volatils, le protocole de mesure est le même que celui décrit précédemment.

Equations utilisées :

Soit 100mg d'une composition comprenant i solvants ayant chacun une vitesse d'évaporation $v_i$ (mesurée selon le protocole précédemment décrit, exprimées en mg d'huile évaporée par unité de surface (cm2) et par unité de temps (minute).

Les solvants sont introduits dans la composition en une quantité initiale par unité de surface égale à $m_i(0)$ (exprimées en mg par cm2).

Pour chaque solvant, la masse restante par unité de surface à un temps t [$m_i(t)$] peut être donnée par les équations suivantes :

$$m_i(t) = m_i(0) - v_i.t \quad si \quad t < \frac{m_i(0)}{v_i}$$

$$m_i(t) = 0 \quad si \quad t \geq \frac{m_i(0)}{v_i}$$

La masse totale de phase grasse liquide peut alors être donnée par la somme de toutes les masses individuelles $m_i(t)$ à chacun des temps :

$$M = \sum_i m_i(t)$$

Le calcul est ainsi effectué pour un temps t = 30 minutes.

On notera que dans cette approche, les huiles non volatiles sont considérées comme ayant des vitesses d'évaporation nulles.

Selon un mode de réalisation avantageux, lorsque l'ester d'acide carbonique est utilisé en mélange avec d'autres solvants lipophiles volatils, il doit être présente à au moins 30%, ou mieux 50% en masse de la somme totale des volatils lipophiles.

Selon un mode de réalisation avantageux, que le ou les esters d'acide carbonique selon l'invention soient utilisés en mélange avec d'autres solvants ou pas, le(s) ester(s) d'acide carbonique selon l'invention représente(nt) de préférence au moins 2% en poids, ou mieux au moins 5% en poids par rapport au poids total de la composition.

Par lipophile, on entend solvants non miscibles à l'eau. On définit les solvants lipophiles à partir de leur paramètre de solubilité δa qui est donné à par la relation suivante :

$$\delta_a = \sqrt{\delta_p^2 + \delta_h^2}$$

où δp et δh sont les paramètres de solubilité de Hansen calculés par contributions de groupe, selon la référence « Van Krevelen, D.W., Properties of Polymer: Their Correlation with Chemical Structure; Their Estimation and Prediction from Additive Group Contribution. 3$^{rd}$ ed. Elsevier (1990) ». Les calculs sont donnés dans le chapitre 7 dudit ouvrage. Les équations donnant les paramètres de solubilité $\delta_d$, $\delta_p$ et $\delta_h$ sont données page 212 (méthode Hoftyzer & Van Krevelen). Elles se calculent à partir du volume molaire du constituant recherché, lequel est donné par la table 7.9 p. 215 ($V = \Sigma N_i V_i$) et des valeurs des $V_i$ figurant dans la table 7.3 p. 196-197. Le nombre $N_i$ représente le nombre de groupe i par molécule. Les équations font également intervenir des paramètres $F_{di}$, $F_{pi}$ et $E_{hi}$, qui sont donnés par la table 7.8 p. 213.

On considère les solvants lipophiles selon la présente invention comme ayant des valeurs de δa < 15 J$^{1/2}$.cm$^{-3/2}$, mieux δa < 10 J$^{1/2}$.cm$^{-3/2}$.

Par "huile volatile" ou "solvant volatil", on entend une huile (ou milieu non aqueux) susceptible de s'évaporer au contact de la peau ou de la fibre kératinique, et plus généralement de la matière kératinique, en moins d'une heure, à température ambiante et pression atmosphérique. L'huile volatile est une huile cosmétique volatile, liquide à température ambiante, ayant notamment une pression de vapeur non nulle, à température ambiante et pression atmosphérique, notamment ayant une pression de vapeur allant de 0,13 Pa à 40 000 Pa (10$^{-3}$ à 300 mm de Hg), en particulier allant de 1,3 Pa à 13 000 Pa (0,01 à 100 mm de Hg), et plus particulièrement allant de 1,3 Pa à 8000 Pa (0,01 à 60 mm de Hg).

Dans le cadre de la présente invention, les huiles volatiles ne répondant pas à la formule (I) et pouvant être présentes dans la composition sont les huiles pour lesquelles la quantité évaporée au bout de 30 minutes dans les conditions décrites ci-avant est supérieure ou égale à 0.07 mg/cm$^2$.

Parmi ces huiles volatiles non-conformes à la formule (I), on peut citer les huiles volatiles siliconées cycliques ou non cycliques, ou les huiles volatiles non siliconées, notamment choisies parmi les huiles volatiles hydrocarbonées ou fluorées, ainsi que leurs mélanges.

Parmi les "huiles volatiles siliconées cycliques ou non cycliques", on peut notamment citer les linéaires ayant une viscosité ≤ 6 centistokes (6.10$^{-6}$ m$^2$/s), et ayant notamment de 3 à 10 atomes de silicium, ces silicones comportant éventuellement un ou plusieurs groupes alkyles ou alkoxy ayant de 1 ou 2 atomes de carbone. Dans cette catégorie d'huiles volatiles siliconées utilisables dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

Les huiles volatiles siliconées non cycliques peuvent encore être choisies parmi les huiles volatiles siliconées linéaires ou ramifiées.

L'huile volatile hydrocarbonée non conforme à la formule (I) peut être choisie parmi les huiles volatiles hydrocarbonées ayant de 8 à 16 atomes de carbone et leurs mélanges et notamment les alcanes ramifiés en C$_8$-C$_{16}$ comme les isoalcanes (appelées aussi isoparaffines), l'isododécane (encore appelée 2,2,4,4,6-pentaméthylheptane), l'isodécane, l'isohexadécane et par exemple les huiles vendues sous les noms commerciaux d'Isopars® ou de Permethyls®.

D'autres huiles hydrocarbonées volatiles peuvent être choisies parmi :

1) Les esters de formule R1COOR2 dans laquelle R1 représente un atome d'hydrogène H ou un radical hydrocarboné linéaire ou ramifié et R2 représente un radical hydrocarboné linéaire ou ramifié, avec le proviso que

- lorsque R2 est un radical hydrocarboné linéaire et R1 est H ou un radical hydrocarboné linéaire, alors 7 ≤ R1 + R2 ≤ 8 ; et
- lorsqu'au moins l'un de R1 et R2 est un radical hydrocarboné ramifié, alors 8 ≤ R1 + R2 ≤ 10.

2) Les cétones de formule R1-CO-R2 dans laquelle R1 et R2 sont identiques ou différents et représentent un radical hydrocarboné linéaire ou ramifié, avec le proviso que :

- lorsque R1 et R2 sont linéaires, ils contiennent chacun de 1 à 8 atomes de carbone avec 8 ≤ R1 + R2 ≤ 9 ; et

- lorsque R1 et/ou R2 sont ramifiés, ils contiennent chacun de 1 à 10 atomes de carbone avec $9 \leq R1 + R2 \leq 11$.

3) Les éthers de formule R1-O-R2, dans laquelle R1 et R2 sont identiques ou différents et représentent un radical hydrocarboné linéaire ou ramifié, avec le proviso que :

- lorsque R1 et R2 sont linéaires, ils contiennent chacun de 1 à 10 atomes de carbone avec $10 \leq R1 + R2 \leq 11$ ; et
- lorsque R1 et/ou R2 sont ramifiés, ils contiennent chacun de 1 à 12 atomes de carbone avec $10 \leq R1 + R2 \leq 13$.

4) Les aldéhydes de formule R1COH, dans laquelle R1 représente un radical hydrocarboné linéaire ou ramifié, avec le proviso que :

- lorsque R1 est linéaire, il contient 7 ou 8 atomes de carbone ; et
- lorsque R1 est ramifié, il contient 8 à 10 atomes de carbone.

Dans ces différentes formules 1) à 4), R1 et R2 (lorsqu'il existe) sont choisis indépendamment l'un de l'autre et sont deux radicaux distincts, c'est-à-dire qu'ils ne sont pas liés l'un à l'autre par une liaison covalente. De façon préférée, R1 et R2 sont des radicaux alkyles.

D'autres huiles hydrocarbonées volatiles comme le distillat de pétrole, notamment ceux vendus sous la dénomination "SHELL SOLT®" par la société SHELL, peuvent être utilisées.

Selon une variante de l'invention, la composition de la présente invention est exempte d'huiles volatiles siliconées, cycliques ou non cycliques, c'est-à-dire comprend moins de 0,1 % en poids de ces huiles volatiles siliconées, cycliques

ou non cycliques, par rapport au poids total de la composition.

Selon une autre variante de l'invention, la composition est exempte d'huile volatile siliconée cyclique, notamment l'octaméthyl cyclotétrasiloxane, le décaméthyl cyclopentasiloxane, le dodécaméthyl cyclohexasiloxane, et en particulier l'octaméthyl cyclotétrasiloxane, c'est à dire comprend moins de 0,1 % en poids de telles huiles siliconées cycliques par rapport au poids total de la composition.

De façon préférée, lorsque l'ester d'acide carbonique de formule (I) est utilisé en mélange avec d'autres solvants volatils non-conformes à la formule (I), ces autres solvants sont naturels ou d'origine naturelle. De façon générale, on préférera que les différentes huiles de la composition (volatiles ou non, et y compris les esters d'acides carbonique de formule (I)), les corps gras solides, ou encore les autres ingrédients de la composition soient naturels ou d'origine naturelle.

Les composés « naturels » sont :

- les composés d'origine végétale agricole biologique ou végétale sauvage avec un prélèvement raisonné,
- les composés d'origine végétale agricole ou provenant du règne protiste,
- les composés d'origine minérale non fossile,
- les composés d'origine animale, de préférence les composés sécrétés par des animaux (cire d'abeille).

Les composés « d'origine naturelle » sont des composés naturels ayant subits des transformations, ces transformations pouvant être :

1) des transformations ne modifiant pas la composition de la matière première par rapport à son origine hormis éventuellement sa teneur en eau. Ces transformations engendrent essentiellement des modifications de l'aspect physique de l'ingrédient par rapport à son origine. Des exemples de transformations entrant dans cette catégorie sont :

- le concassage,
- le broyage,
- le séchage,
- la lyophilisation,
- les procédés thermiques de conservation (appertisation, - pasteurisation),
- les procédés de conservation par pression (Pascalisation),
- l'addition de conservateurs d'origine végétale étant acceptée.

2) des procédés visant à extraire, dans le cas d'ingrédients d'origine végétale, une fraction donnée de la plante sans rupture de liaisons chimiques covalentes, ce qui englobe les procédés suivants :

- l'expression,

- le pressage,
- les procédés de Flash détente,
- la distillation,
- les procédés d'extraction à l'eau (décoction, infusion, macération),
- les procédés d'extraction à l'éthanol (dont l'enfleurage),
- les procédés d'extraction au $CO_2$ super critique,
- les procédés d'extraction ci-dessus utilisant le chauffage par micro-ondes,
- entraînement à la vapeur,
- les procédés de purification,
- les procédés de purification basés sur les technologies précédentes,
- les procédés de purification par passage sur charbon actif, sur oxydes

ou sur résine,

- les procédés thermiques de conservation (appertisation, pasteurisation),
- les procédés de winterisation ou de frigélisation,
- les biotransformations appliquées aux matières premières d'origine végétale et catalysées par des organismes génétiquement non modifiés et dont la fonction d'origine correspond à la réaction ciblée,
- les procédés de conservation par pression (Pascalisation) ou par addition de conservateurs d'origine végétale.
- Les procédés d'extraction génétique ne rentrent pas dans cette catégorie ainsi que les procédés de conservation par irradiation.

3) Dans le cas des matières minérales non fossiles, les procédés de transformation peuvent être les suivants :

- les procédés visant à purifier ou modifier légèrement la matière première sans modification significative de sa structure cristalline ou de sa composition,
- la distillation,
- les procédés de purification (élimination de métaux lourds, de composés organiques...),
- les procédés d'échanges ioniques,
- les procédés de purification par passage sur charbon actif, sur oxydes

ou sur résine,

- les procédés thermiques de conservation,
- les procédés de conservation par pression (Pascalisation),

4) des transformations par procédé chimique engendrant une modification mineure, et notamment en ce qui concerne les composés d'origine végétale :

- extraction par solvant organique (hexane, Ethers fluorés, ou autre),
- hydrolyse,
- estérification,
- oxydation utilisant l'oxygène comme oxydant,
- les hydrogénations d'oléfines,
- les hydrogénations d'acides et d'esters,
- les étherifications,
- la réaction de Guerbet (réaction intermoléculaire entre alcool s'apparentant à un procédé de "cuisson"),

et pour les ingrédients d'origine minérale non fossile : les procédés permettant d'obtenir des matériaux par dissolution re-précipitation d'espèces minérales conduisant à des oxydes simples ou structurés (zéolites, mésoporeux...),

5) des transformations pour une pour une fonctionnalisation, notamment l'amination, la nitration, la sililation, la carboxylation en utilisant des catalyseurs d'origine minérale ou biologique, ainsi que les bio transformations par des organismes génétiquement modifiés dont la fonction correspond ou non à la réaction originelle, et les procédés donnant lieu à la synthèse de mélanges d'oxydes.

Selon un premier mode de réalisation de l'invention, on considère notamment qu'un composé est naturel ou d'origine naturelle comme défini ci-avant aux points 4) ou 5) lorsque la quantité pondérale d'un composé naturel ou d'origine naturelle est supérieure à la quantité pondérale qui ne répond pas à cette définition.

Selon un second mode de réalisation, on considère on considère notamment qu'un composé est naturel ou d'origine naturelle comme défini ci-avant aux points 4) ou 5) lorsque le nombre d'atomes de carbone d'un composé naturel ou d'origine naturelle est supérieur au nombre d'atomes de carbone qui ne répond pas à cette définition.

Ainsi, ne sont donc pas considérés comme des composés naturels ou d'origine naturelle certains solvants volatils conventionnellement utilisés dans les compositions cosmétiques tels que l'isododécane, qui est d'origine minérale fossile (issu de la chimie du pétrole) ou la cyclométhicone D5 qui est un composé siliconé préparé par des procédés de synthèse chimique.

De façon avantageuse, les compositions selon l'invention sont telles que les solvants volatils qui ne sont pas naturels ou d'origine naturelle représentent moins de 20% en masse de la somme des solvants volatils de la composition.

De façon préférée, la composition est telle que le mélange desdits esters d'acide carbonique et/ou desdites huiles volatiles non-conforme(s) à la formule (I) et/ou de corps gras éventuellement présents contient moins de 2% en masse de composés non naturels ou n'étant pas d'origine naturelle, par rapport à la masse dudit mélange (ledit mélange pouvant ainsi être totalement exempt de tels composés).

Selon un mode de réalisation particulier, lorsque l'ester d'acide carbonique de formule (I) est utilisé en mélanges avec d'autres solvants volatils lipophiles ne répondant pas à la formule (I), le mélange doit être réalisé de telle façon que le mélange de solvants volatils, ou phase grasse volatile, dans la composition selon l'invention présente un profil d'évaporation tel que la masse d'huile(s) évaporée(s) au bout de 30 minutes selon les conditions définies ci-dessus est comprise entre 4.1 mg/cm$^2$ et 24 mg/cm$^2$.

De façon préférée, la phase grasse volatile comprenant les esters d'acide carbonique selon la formule (I) et éventuellement d'autres huiles volatiles représente une teneur variant de 0,1 à 80 % en poids, notamment de 1 à 65 % en poids, en particulier de 10 à 50 % en poids, par rapport au poids total de la composition.

Selon un autre aspect, l'invention a également pour objet l'utilisation des esters d'acide carbonique de formule (I) en tant que solvant volatil pour la préparation d'une composition cosmétique.

Selon un autre aspect, l'invention a encore pour objet un procédé cosmétique de maquillage et/ou de soin des matières kératiniques comprenant au moins l'étape d'application sur les matières kératiniques d'une composition selon l'invention.

Un autre objet de l'invention est un procédé de préparation de telles compositions de maquillage et/ou de soin.

## Milieu physiologiquement acceptable

Par « milieu physiologiquement acceptable », on désigne un milieu non toxique et susceptible d'être appliqué sur la peau, notamment du corps, des mains, du cou, du visage, les lèvres et/ou les fibres kératiniques, d'êtres humains. Le milieu physiologiquement acceptable est généralement adapté à la nature du support sur lequel doit être appliquée la composition ainsi qu'à l'aspect sous lequel la composition est destinée à être conditionnée.

## Huiles non volatiles

La composition selon l'invention peut en outre comprendre au moins une huile non volatile. Celle-ci peut être en particulier choisie parmi les huiles hydrocarbonées et/ou siliconées et/ou fluorées non volatiles.
On entend par « huile non volatile », une huile restant sur la peau ou la fibre kératinique, plus généralement sur la matière kératinique, à température ambiante et pression atmosphérique, au moins plusieurs heures et ayant notamment une pression de vapeur inférieure à 10$^{-3}$ mm de Hg (0,13 Pa). On peut également définir une huile non volatile comme ayant une vitesse d'évaporation telle que dans les conditions définies précédemment, la quantité évaporée au bout de 30 minutes est inférieure à 0,07mg/cm$^2$.

Comme huile hydrocarbonée non volatile, on peut notamment citer :

- les huiles hydrocarbonées d'origine végétale telles que les triglycérides constitués d'esters d'acides gras et de glycérol dont les acides gras peuvent avoir des longueurs de chaînes variées de C$_4$ à C$_{24}$, ces derniers pouvant être linéaires

ou ramifiées, saturées ou insaturées, comme les triglycérides des acides heptanoïque, octanoïque ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations de « Miglyol 810® » , « 812® » et « 818® » par la société Dynamit Nobel,

- les huiles d'origine animale comme l'huile de vison, de tortue, le perhydrosqualène,
- les éthers de synthèse ;
- les hydrocarbures linéaires ou ramifiés, d'origine minérale ou synthétique tels que l'huile de paraffine ou ses dérivés, la vaseline, les polydécènes, le polyisobutène hydrogéné tel que le Parléam® commercialisé par la société NIPPON OIL FATS, le squalane, et leurs mélanges;
- les esters d'acide gras, en particulier de 4 à 22 atomes de carbone, et notamment d'acide octanoïque, d'acide heptanoïque, d'acide lanolique, d'acide oléique, d'acide laurique, d'acide stéarique comme le dioctanoate de propylène glycol, le monoisostéarate de propylène glycol, le poly-glycéryl 2-diisostéarate, le diheptanoate de néopentylglycol,
- les esters de synthèse comme les huiles de formule $R_1COOR_2$ dans laquelle $R_1$ représente le reste d'un acide gras linéaire ou ramifié comportant de 1 à 40 atomes de carbone et $R_2$ représente une chaîne hydrocarbonée notamment ramifiée contenant de 1 à 40 atomes de carbone à condition que $R_1 + R_2$ soit $\geq$ 11, comme par exemple l'huile de Purcellin (octanoate de cétostéaryle), l'isononanoate d'isononyle, le benzoate d'alcool en $C_{12}$ à $C_{15}$, le palmitate d'éthyl 2-hexyle, le stéarate d'octyl-2 dodécyle, l'érucate d'octyl-2 dodécyle, l'isostéarate d'isostéaryle, le benzoate d'octyl-2 dodécyle, des octanoates, décanoates ou ricinoléates d'alcools ou de polyalcools, le myristate d'isopropyle, le palmitate d'isopropyle, le stéarate de butyle, le laurate d'hexyle, l'adipate de diisopropyle, le palmitate de 2-éthylhexyle, le laurate de 2-hexyl-décyle, le palmitate de 2-octyldécyle, le myristate de 2-octyldodécyle, le succinate de 2-diéthyl-hexyle, le malate de diisostéaryle, le néopentanoate d'isodécyle ;
- les esters hydroxylés comme le lactate d'isostéaryle, l'octylhydroxystéarate, l'hydroxystéarate d'octyldodécyle, le diisostéarylmalate, le citrate de triisocétyle, le triisostéarate de glycérine ou de diglycérine; le diisononanoate de diéthylèneglycol ; et
- les esters du pentaérythritol ; les esters d'acides aromatiques et d'alcools comprenant 4 à 22 atomes de carbone, notamment le trimellitate de tridécyle,
- les alcools gras liquides à température ambiante à chaîne carbonée ramifiée et/ou insaturée ayant de 8 à 26 atomes de carbone comme l'alcool oléique, l'alcool linoléique ou linolénique, l'alcool isostéarique ou l'octyl dodécanol; les acides gras supérieurs en $C_8$-$C_{26}$ tels que l'acide oléique, l'acide linoléique, l'acide linolénique, ou l'acide isostéarique ;
- et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes ou les 2-phényléthyl triméthylsiloxysilicates.

Selon un aspect de l'invention, la composition est exempte d'huile non volatile, c'est-à-dire comprend moins de 0,1 % en poids d'huile non volatile par rapport au poids total de la composition.

Selon un autre aspect de l'invention, l'huile non volatile peut être présente en une teneur allant de 0,1 à 60 % en poids, notamment allant de 0,5 à 50 % en poids, et en particulier allant de 1 à 40 % en poids, par rapport au poids total de la composition.

### Corps gras solides

La composition selon l'invention peut comprendre, en particulier lorsqu'il s'agit d'un rouge à lèvres ou d'un fond de teint, au moins un corps gras solide à température ambiante et à pression atmosphérique, il peut être choisi parmi les cires, les corps gras pâteux, les gommes et leurs mélanges. Ce corps gras solide peut être présent dans une teneur variant de 0,01 à 60, notamment de 0,1 à 50 % et en particulier de 0,1 à 40 % en poids, par rapport au poids total de la composition.

Ainsi, la composition selon l'invention peut comprendre au moins un composé gras pâteux à température ambiante.

Par "corps gras pâteux" au sens de l'invention, on entend des corps gras ayant un point de fusion allant de 20 à 55 °C, en particulier 25 à 45°C et/ou une viscosité à 40 °C allant de 0,1 à 40 Pa.s (1 à 400 poises), en particulier 0,5 à 25 Pa.s, mesurée au Contraves TV ou Rhéomat 80, équipé d'un mobile tournant à 60 Hz. L'homme du métier peut choisir le mobile permettant de mesurer la viscosité, parmi les mobiles MS-r3 et MS-r4, sur la base de ses connaissances générales, de manière à pouvoir réaliser la mesure du composé pâteux testé.

Plus particulièrement, ces corps gras peuvent être des composés hydrocarbonés, éventuellement de type polymérique ; ils peuvent également être choisis parmi les composés siliconés; ils peuvent aussi se présenter sous forme d'un mélange de composés hydrocarbonés et/ou siliconés. Dans le cas d'un mélange de différents corps gras pâteux, on utilise de préférence les composés pâteux hydrocarbonés (contenant principalement des atomes de carbone et d'hydrogène et éventuellement des groupements ester), en proportion majoritaire.

Parmi les composés pâteux susceptibles d'être utilisés dans la composition selon l'invention, on peut citer les lanolines et les dérivés de lanoline comme les lanolines acétylées, les lanolines oxypropylènées ou le lanolate d'isopropyle, ayant une viscosité de 18 à 21 Pa.s, de préférence 19 à 20,5 Pa.s, et/ou un point de fusion de 30 à 55°C et leurs mélanges. On peut également utiliser des esters d'acides ou d'alcools gras, notamment ceux ayant 20 à 65 atomes de carbone (point de fusion de l'ordre de 20 à 35°C et/ou viscosité à 40 °C allant de 0,1 à 40 Pa.s) comme le citrate de triisostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux et leurs mélanges. Comme triglycéride d'origine végétale, on peut utiliser les dérivés d'huile de ricin hydrogénée, tels que le « THIXINR® » de Rheox.

On peut aussi citer les corps gras pâteux siliconés tels que les polydiméthylsiloxanes (PDMS) de hauts poids moléculaires et en particulier ceux ayant des chaînes pendantes du type alkyle ou alcoxy ayant de 8 à 24 atomes de carbone, et un point de fusion de 20-55°C, comme les stéaryl diméthicones notamment ceux vendus par la société Dow Corning sous les noms commerciaux de DC2503® et DC25514® et leurs mélanges.

Le corps gras pâteux peut être présent dans la composition selon l'invention en une teneur allant de 0,01 à 50% en poids, notamment allant de 0,1 à 45 % en poids, et en particulier allant de 0,2 % à 30 % en poids, par rapport au poids total de la composition.

Par « cire » dans le cadre de la présente invention, on entend généralement un composé lipophile, solide à température ambiante (25 °C), déformable ou non déformable, à changement d'état solide/liquide réversible, ayant un point de fusion supérieur ou égal à 30 °C pouvant aller jusqu'à 200 °C et notamment jusqu'à 120 °C.

En portant la cire à l'état liquide (fusion), il est possible de la rendre miscible aux huiles et de former un mélange homogène microscopiquement, mais en ramenant la température du mélange à la température ambiante, on obtient une recristallisation de la cire dans les huiles du mélange.

En particulier, les cires convenant à l'invention peuvent présenter un point de fusion supérieur ou égal à 45 °C, et en particulier supérieur ou égal à 55°C.

Au sens de l'invention, la température de fusion correspond à la température du pic le plus endothermique observé en analyse thermique (DSC) telle que décrite dans la norme ISO 11357-3 ; 1999. Le point de fusion de la cire peut être mesuré à l'aide d'un calorimètre à balayage différentiel (DSC), par exemple le calorimètre vendu sous la dénomination « MDSC 2920 » par la société TA Instruments.

Le protocole de mesure est le suivant :

Un échantillon de 5 mg de cire disposé dans un creuset est soumis à une première montée en température allant de -20 °C à 100 °C, à la vitesse de chauffe de 10 °C/minute, puis est refroidi de 100 °C à -20 °C à une vitesse de refroidissement de 10 °C/minute et enfin soumis à une deuxième montée en température allant de -20 °C à 100 °C à une vitesse de chauffe de 5 °C/minute. Pendant la deuxième montée en température, on mesure la variation de la différence de puissance absorbée par le creuset vide et par le creuset contenant l'échantillon de cire en fonction de la température. Le point de fusion du composé est la valeur de la température correspondant au sommet du pic de la courbe représentant la variation de la différence de puissance absorbée en fonction de la température.

A titre de cires pouvant être utilisées selon l'invention, on peut citer :

- les cires d'origine animale telles que la cire d'abeilles, la cire de lanoline et les dérivés de lanoline, les cires végétales telles que la cire de Carnauba, de Candellila, d'Ouricury, du Japon, le beurre de cacao ou les cires de fibres de liège ou de canne à sucre,
- les cires minérales, par exemple de paraffine, de vaseline, de lignite ou les cires microcristallines ou les ozokérites,
- les cires synthétiques parmi lesquelles les cires de polyéthylène, et les cires obtenues par synthèse de Fisher-Tropsch,
- les cires de silicone, en particulier les polysiloxanes linéaires substitués; on peut citer, par exemple, les cires de silicone polyéther, les alkyl ou alkoxydiméthicones ayant de 16 à 45 atomes de carbone, les alkyl méthicones comme la $C_{30}$-$C_{45}$ alkyl méthicone vendue sous la dénomination commerciale "AMS C 30" par DOW CORNING,
- les huiles hydrogénées concrètes à 25°C telles que l'huile de ricin hydrogénée, l'huile de jojoba hydrogénée, l'huile de palme hydrogénée, le suif hydrogéné, l'huile de coco hydrogénée et les esters gras concrets à 25°C comme le stéarate d'alkyle en $C_{20}$-$C_{40}$ vendu sous la dénomination commerciale "KESTER WAX K82H" par la société KOSTER KEUNEN,
- et/ou leurs mélanges.

De préférence, on utilisera les cires de polyéthylène, les cires microcristallines, les cires de carnauba, l'huile de jojoba hydrogénée, les cires de candellila, les cires d'abeilles et/ou leurs mélanges.

**Phase aqueuse et/ou hydrosoluble**

La composition selon l'invention peut comprendre en outre au moins une phase aqueuse contenant de l'eau. L'eau peut être une eau florale telle que l'eau de bleuet et/ou une eau minérale telle que l'eau de VITTEL, l'eau de LUCAS ou l'eau de LA ROCHE POSAY et/ou une eau thermale.

La phase aqueuse peut également comprendre des solvants organiques miscibles à l'eau (à 25 °C) comme par exemple les alcools primaires tels que l'éthanol et l'isopropanol, les glycols tels que le glycérol, le propylène glycol, le butylène glycol, le dipropylène glycol, le diéthylène glycol, les éthers de glycol, les alkyl en $C_1$ à $C_4$ éthers de mono-, di- ou tri- propylène glycol, mono-, di- ou triéthylène glycol, et leurs mélanges.

La composition peut être une composition anhydre, c'est-à-dire une composition contenant moins de 2 % en poids d'eau, voire moins de 0,5 % d'eau, notamment exempte d'eau, l'eau n'étant pas ajoutée lors de la préparation de la composition mais correspondant à l'eau résiduelle apportée par les ingrédients mélangés.

**Phase particulaire**

La composition de l'invention, peut en outre comprendre, en particulier lorsqu'il s'agit d'un rouge à lèvres ou d'un fond de teint, une phase particulaire additionnelle pouvant être présente à raison de 0,01 à 50 % en poids, notamment de 0,01 à 40 % en poids et en particulier de 0,05 à 30 % en poids, par rapport au poids total de la composition.

Elle peut notamment comprendre des pigments et/ou des nacres et/ou des charges complémentaires utilisés dans les compositions cosmétiques.

Selon un mode de réalisation, la composition selon l'invention comprend au moins un pigment.

Par pigments, il faut comprendre des particules blanches ou colorées, minérales ou organiques, insolubles dans la phase hydrophile liquide, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées.

Les pigments peuvent être présents dans la composition à raison de 0,01 à 25 % en poids, en particulier de 0,01 à 20 % en poids, et notamment de 0,02 à 15 % en poids par rapport au poids de la composition.

Comme pigments minéraux utilisables dans l'invention, on peut citer les oxydes de titane, de zirconium ou de cérium ainsi que les oxydes de zinc, de fer ou de chrome, le bleu ferrique, le violet de manganèse, le bleu outremer et l'hydrate de chrome. Parmi les pigments organiques utilisables dans l'invention, on peut citer le noir de carbone, les pigments de type D & C, et les laques à base de carmin de cochenille, de baryum, strontium, calcium, aluminium ou encore les dicéto pyrrolopyrrole (DPP) décrits dans les documents EP-A-542669, EP-A-787730, EP-A-787731 et WO-A- 96/08537.

Les nacres peuvent être présentes dans la composition à raison de 0,01 à 25 % en poids, notamment de 0,01 à 15 % en poids, et en particulier de 0,02 à 10 % en poids, par rapport au poids total de la composition.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Selon un mode de réalisation, la composition selon l'invention comprend au moins une charge.

Les charges complémentaires peuvent être présentes à raison de 0, 01 à 50 % en poids, notamment 0,01 à 40 % en poids, et en particulier de 0,02 % à 30 % en poids et encore en particulier 0,02 % à 20 % en poids de par rapport au poids total de la composition.

Il peut notamment s'agir de charges sphériques comme par exemple le talc, le stéarate de zinc, le mica, le kaolin, les poudres de polyamide (Nylon®) (Orgasol® de chez Atochem), les poudres de polyéthylène, les poudres de polymères de tétrafluoroéthylène (Téflon®), l'amidon, le nitrure de bore, des microsphères polymériques telles que celles de chlorure de polyvinylidène/acrylonitrile comme l'Expancel® (Nobel Industrie), de copolymères d'acide acrylique (Polytrap® de la société Dow Corning), les microbilles de résine de silicone (Tospearls® de Toshiba, par exemple), et les organopolysiloxanes élastomères.

La composition peut comprendre également des colorants hydrosolubles ou liposolubles en une teneur allant de 0,01 à 6 % en poids, par rapport au poids total de la composition, notamment allant de 0,01 à 3 % en poids. Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, et le jaune quinoléine. Les colorants hydrosolubles sont par exemple le jus de betterave et le bleu de méthylène.

**Matière colorante**

De façon préférée, la composition selon l'invention comprend au moins une matière colorante. Par « matière colorante », on entend les pigments et/ou les colorants et/ou les nacres, et/ou leurs mélanges, tels que définis précédemment.

Les matières colorantes peuvent être présentes dans la composition en une teneur allant de 0,01% à 50% en poids, par rapport au poids total de la composition, de préférence de 0,01% à 30% en poids.

**Additifs**

La composition selon l'invention peut, de plus, comprendre tous les ingrédients classiquement utilisés dans les domaines concernés et plus spécialement dans le domaine cosmétique et dermatologique. Ces ingrédients peuvent notamment être choisis parmi les polymères, notamment les polymères filmogènes, les polymères fixants ; les tensioactifs ; les agents conditionneurs de cheveux ; les opacifiants ; les parfums ; les épaississants ; les gélifiants ; les colorants capillaires ; les résines de silicone ; les gomme de silicone ; les conservateurs ; les antioxydants, les actifs cosmétiques ; les filtres solaires ; les agents de stabilisation du pH ; les vitamines ; les hydratants ; les agents antitranspirants ; les agents déodorants ; les composés autobronzants et leurs mélanges. Les quantités de ces différents ingrédients sont celles classiquement utilisées dans les domaines concernés et par exemple de 0,01 à 20 % du poids total de la composition.

Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction considérée.

La composition de l'invention peut être obtenue selon les procédés de préparation classiquement utilisés en cosmétique ou en dermatologie.

Les exemples suivants sont donnés à titre illustratif et sans caractère limitatif de l'invention.

**Formulation**

La composition selon l'invention peut se présenter sous forme liquide, pâteuse, solide, de mousse ou de spray. Il peut s'agir d'une émulsion, notamment directe ou inverse, ou encore d'une composition anhydre. Elle peut également être sous une forme biphasique.

La composition trouve une application particulière comme composition de soin du corps ou du visage, composition de nettoyage du corps ou du visage telle que gel-douche, gel de bain, démaquillant ; composition de maquillage du corps ou du visage telle que fond de teint, rouge à lèvres, soin pour lèvres, soin des ongles, mascara, eye-liner ; composition parfumante ; composition capillaire telle que composition de coloration des cheveux, composition de déformation permanente des cheveux ; composition de protection solaire ; composition déodorante ; composition de nettoyage ou de soin des cheveux telle que shampooing, après-shampooing à rincer ou non, composition à rincer à appliquer avant ou après une coloration, une décoloration, une permanente ou un défrisage ou encore entre les deux étapes d'une permanente ou d'un défrisage ; composition capillaire pour le maintien de la coiffure telle qu'une laque, un gel, une mousse ou un spray de coiffage.

En particulier, la composition selon l'invention peut être utilisée pour le maquillage et/ou le soin de la peau, des lèvres et/ou des fibres kératiniques d'être humain.

Selon un aspect préféré de l'invention, la composition est sous la forme de rouges à lèvres ou de produits du teint, notamment du type fond de teint, ou encore de mascara.

Lorsque la composition selon l'invention est du type mascara, celle-ci peut être appliquée de manière homogène ou non homogène à la surface des cils, en une couche unique ou sous la forme de plusieurs couches superposées. La composition selon l'invention peut alors être plus particulièrement destinée à un produit de mascara comprenant un réservoir, contenant au moins ladite composition de mascara, et un système d'application de ladite composition sur les fibres kératiniques, comme les cils.

Selon un aspect de l'invention, cette composition se présente sous la forme d'un produit coulé en stick ou en coupelle comme les rouges à lèvres ou les baumes à lèvres, les fonds de teint coulés, les produits anti-cernes, les « correcteurs » et/ou « embellisseurs » de teint et les fards à paupières ou à joues.

Au sens de la présente invention, on entend qualifier par « composition coulée », toute composition cosmétique dénuée de la faculté d'écoulement sous l'action de son propre poids, par opposition aux compositions dites fluides.

Ces compositions peuvent, le cas échéant, présenter un aspect pâteux à température ambiante (25°C). Ainsi une composition cosmétique selon l'invention peut posséder un point de fusion ou une température de transition thermique telle qu'un point de ramollissement, supérieure à 25°C, notamment pouvant varier de 25 à 85°C, voire de 30 à 60°C, et en particulier de 30 à 45°C et/ou une dureté pouvant varier de 0,001 à 0,5MPa, notamment de 0,005 à 0,4MPa.

Les compositions selon cet aspect de l'invention, à savoir de type coulée, présentent des duretés, notamment lorsqu'elles se présentent sous forme de stick.

Les exemples qui suivent ont pour but d'illustrer de manière non limitative l'objet de la présente invention. Les quantités sont données en pourcentage massique.

**Exemple 1 :**

Fond de teint sous forme d'émulsion eau-dans-huile ayant la composition suivante:

| | |
|---|---|
| Cétyl diméthicone copolyol (ABIL EM 90 de la société GOLDSCHMIDT) | 3 g |
| Succinate d'isostéaryl diglycéryle (IMWITOR 780K de la société CONDEA) | 0,6 g |
| Carbonate de méthyle et de n-pentyle | 23.58g |
| Mélange de pigments (oxydes de fer et oxydes de titane hydrophobes) | 10 g |
| Bentone | 1,6 g |
| Poudre de polyamide (NYLON-12 de Dupont de Nemours) | 8 g |
| Sulfate de magnésium | 0,7 g |
| Conservateur | 0,45 g |
| Parfum | 0,5 g |
| Eau | qsp 100 g |

**Exemple 2 :**

Fond de teint huile-dans-eau ayant la composition suivante :

| | |
|---|---|
| Carbonate de n-hexyle et de méthyle | 11 g |
| Polyisobutène hydrogéné (Parleam, NOF COrporation) | 5 g |
| Palmitate d'éthyl-2 hexyle | 11 g |
| Isostéarate de glycéryle | 4 g |
| Acide stéarique | 2 g |
| Triéthanolamine | 1 g |
| Poudre de polyamide (NYLON-12 de Dupont de Nemours) | 5 g |

| | |
|---|---|
| Mélange de pigments (oxydes de fer et oxydes de titane) | 10 g |
| Carboxyméthylcellulose | 0,2 g |
| Propylène glycol | 5g |
| Glycérol | 2g |
| Parfum | 0,5g |
| Conservateurs | 0,4g |
| Eau | qsp 100 g |

**Exemple 3 :**

Rouge à lèvres ayant la composition suivante :

| | |
|---|---|
| Cire de polyéthylène (Performalene 655, New Phase Technologies) | 15g |
| Carbonate de dipropyle (294934-25G, Aldrich®) | 70g |
| Carbonate d'éthyle et de n-pentyle | 9 g |
| DC Red n°7 Calcium Lake (pigment) | 6 g |

**Exemple 4 :**

Crème de soin ayant la composition suivante :

Phase grasse

| | |
|---|---|
| Mélange de monostéarate de glycéryle et de stéarate de polyéthylène glycol 100 OE (50/50 en poids) (ARLACEL 165 de la société ICI) | 2,5 g |
| Alcool stéarylique | 0,5 g |
| Acide stéarique | 1 g |
| Polyisobutène hydrogéné (Parleam, NOF COrporation) | 9 g |
| Carbonate de n-butyle et d'éthyle | 4.2g |

Phase aqueuse

| | |
|---|---|
| Acide polyacrylique réticulé (Carbopol 980) | 1 g |
| Triéthanolamine | 0,03 g |
| Conservateur | 0,3 g |
| Eau | qsp 100 g |

**Exemple 5 :**

Démaquillant ayant la composition suivante :

| | |
|---|---|
| Palmitate d'isopropyle | 8 g |
| Carbonate de dipropyle (294934-25G, Aldrich ®) | 8g |
| Alcool stéarique | 8 g |
| Stéarate de sucrose | 2 g |
| Acide stéarique | 0,3 g |
| Soude | 0,06 g |
| Glycérine | 5 g |
| Carbopol | 0,2 g |
| Eau | qsp 100 g |

**Exemple 6 :**

Déodorant en spray ayant la composition suivante :

| | |
|---|---|
| Carbonate de dipropyle (294934-25G, Aldrich ®) | 33g |
| PPG-14 butyl ether (Ucon Fluid AP - Amerchol) | 10g |
| Huile de ricin hydrogénée (Cutina HR - Cognis) | 4g |
| Talc | 2g |
| Alulinium chlorhydrate (Micro Dry - Reheis) | 20g |
| Alcool stéarylique | 14g |
| PEG-8 distearate (distéarate de PEG 400 - Stéarineries Dubois) | 2g |
| C12-15 alkyl benzoate (Finsolv TN - Witco) | 15g |

**Exemple 7 :**

Déodorant roll-on (émulsion) ayant la composition suivante :

| | |
|---|---|
| Aluminium Chrlorhydrate (50% solution) (Chlorhydrol 50% USP) | 40g |
| Steareth 21 (Brij 721 - ICI) | 2g |
| Steareth 2 (Brij 2 - ICI) | 2g |
| PPG 15 stearyl ether (Arlamol E - ICI) | 1.5g |
| Carbonate d'éthyle et de n-pentyle | 3.5g |
| Eau | qsp 100g |

**Exemple 8 :**

Aérosol anhydre anti-transpirant ayant la composition suivante :

| | |
|---|---|
| Stearalkonium bentonite vendue sous le nom Tixogel MP250 par Sud-Chemie Rheologicals, United Catalysts Inc. | 0.5g |
| Aluminium Chlorhydrate | 7g |
| Isobutane | 80g |
| Triethylcitrate | 1.4g |
| Palmitate d'isopropyle | 3g |
| Carbonate de 3,3-diméthylbutyle et de méthyle | 8.1 g |

**Exemple 9 :**

Crème solaire ayant la composition suivante :

| | |
|---|---|
| Acide stéarique | 0,95g |
| Glycéryl stéarate (et) PEG-100 Stéarate | 2,00g |
| Cétyl alcohol (et) Myristyl alcohol (et) stéaryl alcohol | 0,50g |
| Diméthicone | 0,50g |
| Phénoxyéthanol (et) Méthylparaben (et) éthylparaben (et) propylparaben (et) isobutylparaben (et) butylparaben | 1,00g |
| C12-15 alkyl benzoate | 8,00g |
| Éthylhexyl cocoate | 2,00g |
| Octocrylène | 7,00g |
| Ethylhexyl triazone | 1,00g |
| Butyl méthoxydibenzoylméthane | 3,50g |
| Triéthanolamine | 0,50g |
| Glycérol | 4,00g |
| Méthylparaben (et) butylparaben (et) éthylparaben (et) isobutylparaben (et) propylparaben | 0,25g |
| Disodium EDTA | 0,10g |
| Eau | 52,10g |
| Carbomer | 0,30g |
| Potassium cétyl phosphate | 1,00g |
| Triéthanolamine | 0,30g |
| Titanium dioxide (et) Aluminium hydroxide (et) acide stéarique | 5,00g |
| Carbonate de n-butyle et de n-propyle | 10.00g |

**Exemple 10 :**

Spray de coiffage en flacon pompe, de composition suivante :

| | |
|---|---|
| Polymère amphotère octylacrylamide/acrylates/ butylaminoéthyl méthacrylate (Amphomer ®, National Starch) | 6 g en MA |
| Carbonate d'isooctyle et de méthyle | 3 g |
| Ethanol | qsp 100 g |

**Revendications**

1. Composition cosmétique comprenant, dans un milieu physiologiquement acceptable, au moins un ester d'acide carbonique répondant à la formule (I) suivante :

$$R1-O-CO-O-R2 \qquad (I)$$

dans laquelle R1 et R2 sont identiques ou différents et représentent un radical hydrocarboné linéaire ou ramifié, avec le proviso que

- lorsque R1 et R2 sont linéaires, ils contiennent chacun de 1 à 6 atomes de carbone avec $6 \leq R1 + R2 \leq 7$ ; et
- lorsque R1 et/ou R2 sont ramifiés, ils contiennent chacun de 1 à 8 atomes de carbone avec $6 \leq R1 + R2 \leq 9$.

2. Composition selon la revendication 1, **caractérisée en ce que** R1 et R2 sont aliphatiques.

3. Composition selon la revendication 2, **caractérisée en ce que** R1 et R2, identiques ou différents, sont un radical alkyl.

4. Composition selon l'une quelconque des revendications précédentes, **caractérisée en ce que** R1 et/ou R2 sont ramifiés.

5. Composition selon l'une quelconque des revendications précédentes, **caractérisée en ce que** l'ester d'acide carbonique de formule (I) ne comprend pas plus de 4 ramifications, ou mieux, pas plus de 2 ou 3 ramifications.

6. Composition selon l'une quelconque des revendications précédentes, **caractérisée en ce que** R1 et/ou R2 ne comprend pas de carbone quaternaire.

7. Composition selon l'une quelconque des revendications 1 à 3, **caractérisée en ce que** R1 et R2 sont linéaires.

8. Composition selon l'une quelconque des revendications précédentes, **caractérisée en ce que** R1 + R2 = 8 atomes de carbone.

9. Composition selon l'une quelconque des revendications 1 à 7, **caractérisée en ce que** R1 + R2 =9 atomes de carbone.

10. Composition selon l'une quelconque des revendications 1à 6, **caractérisée en ce que** R1 + R2 =10 atomes de carbone.

11. Composition selon l'une quelconque des revendications 1 à 6, **caractérisée en ce que** R1 + R2 =11 atomes de carbone.

12. Composition selon l'une quelconque des revendications précédentes, **caractérisée en ce que** l'ester d'acide carbonique de formule (I) est choisi parmi :

- Carbonate de méthyle et de n-pentyle ;
- Carbonate de n-butyle et d'éthyle;
- Carbonate de dipropyle ;
- Carbonate d'éthyle et de n-pentyle ;
- Carbonate de n-butyle et de n-propyle ;
- Carbonate de n-hexyle et de méthyle ;
- Carbonate de 3,3-diméthylbutyle et de méthyle ;
- Carbonate de 1-méthyléthyle et de n-propyle ;
- Carbonate d'éthyle et de 1-éthylpropyle ;
- Carbonate d'éthyle et de 1-méthylpentyle ;
- Carbonate d'isooctyle et de méthyle ;
- Carbonate de 1,1-diéthylpropyle et de méthyle.

13. Composition selon l'une quelconque des revendications précédentes, **caractérisée en ce que** ledit ester d'acide carbonique a une de vitesse d'évaporation telle que la quantité évaporée en 30 minutes est comprise entre 4.1 mg/cm$^2$ et 24 mg/cm$^2$.

14. Composition selon l'une quelconque des revendications précédentes, **caractérisée en ce** ledit ester d'acide carbonique présente un point éclair compris entre 43 et 100°C, et plus particulièrement, entre 45 et 80°C.

15. Composition selon l'une quelconque des revendications précédentes, **caractérisée en ce qu'**elle comprend en outre une ou plusieurs huile(s) volatile(s) non conforme à la formule (I).

16. Composition selon la revendication 15, **caractérisée en ce que** le mélange de solvants volatils a une vitesse d'évaporation telle que la quantité évaporée en 30 minutes est comprise entre 4.1 mg/cm$^2$ et 24 mg/cm$^2$.

17. Composition selon l'une quelconque des revendications précédentes, **caractérisée en ce que** lesdits esters d'acide carbonique et/ou lesdites huiles volatiles non-conforme à la formule (I) sont naturelles ou d'origine naturelle.

18. Composition selon l'une quelconque des revendications précédentes, **caractérisée en ce que** les solvants volatils non naturels ou n'étant pas d'origine naturelle représentent moins de 20% en masse de la somme totale des solvants volatils de la composition.

19. Composition selon l'une quelconque des revendications précédentes, **caractérisée en ce que** le mélange desdits esters d'acide carbonique et/ou desdites huiles volatiles non-conforme(s) à la formule (I) et/ou de corps gras éventuellement présents contient moins de 2% en masse de composés non naturels ou n'étant pas d'origine naturelle, par rapport à la masse dudit mélange.

20. Composition selon l'une quelconque des revendications 15 à 19, **caractérisée en ce que** l'ester d'acide carbonique de formule (I) représente au moins 30 %, et notamment au moins 50%, en masse de la somme totale des solvants volatils de la composition.

21. Composition selon l'une quelconque des revendications précédentes, **caractérisée en ce que** ledit ester d'acide carbonique selon l'invention représente au moins 2% en poids, ou mieux au moins 5% en poids par rapport au poids total de la composition.

22. Composition selon l'une quelconque des revendications précédentes, **caractérisée en ce qu'**elle se présente sous forme liquide, pâteuse, solide, de mousse ou de spray.

23. Composition selon l'une quelconque des revendications précédentes, **caractérisée en ce qu'**elle se présente sous forme d'une émulsion ou d'une composition anhydre.

24. Composition selon l'une quelconque des revendications précédentes, **caractérisée en ce qu'**elle se présente sous la forme d'une composition de soin du corps ou du visage ; composition de nettoyage du corps ou du visage telle que gel-douche, gel de bain, démaquillant ; composition de maquillage du corps ou du visage telle que fond de teint, rouge à lèvres, soin pour lèvres, soin des ongles, mascara, eye-liner ; composition parfumante ; composition capillaire telle que composition de coloration des cheveux, composition de déformation permanente des cheveux ; composition de protection solaire ; composition déodorante ; composition de nettoyage ou de soin des cheveux telle que shampooing, après-shampooing à rincer ou non, composition à rincer à appliquer avant ou après une coloration, une décoloration, une permanente ou un défrisage ou encore entre les deux étapes d'une permanente ou d'un défrisage ; composition capillaire pour le maintien de la coiffure telle qu'une laque, un gel, une mousse ou un spray de coiffage.

25. Composition selon l'une quelconque des revendications précédentes, **caractérisée en ce qu'**elle se présente sous la forme d'un produit coulé en stick ou en coupelle comme les rouge à lèvres ou les baumes à lèvres, les fond de teint coulés, les produits anti-cernes, les "correcteurs" et/ou "embellisseurs" de teint et les fards à paupières ou à joues.

26. Composition selon l'une quelconque des revendications précédentes, **caractérisée en ce qu'**elle comprend au moins une huile non volatile, en particulier choisie parmi les huiles naturelles ou d'origine naturelle.

27. Composition selon l'une quelconque des revendications précédentes, **caractérisée en ce qu'**elle comprend, au moins un corps gras solide à température ambiante et pression atmosphérique.

28. Composition selon la revendication 27, **caractérisée en ce que** le corps gras solide est choisi parmi les cires, les corps gras pâteux, les gommes et leurs mélanges.

29. Composition selon l'une quelconque des revendications précédentes, **caractérisée en ce qu'**elle comprend une phase aqueuse.

**30.** Composition selon l'une quelconque des revendications précédentes, **caractérisée en ce qu'**elle comprend au moins une phase particulaire, comprenant notamment des pigments et/ou des charges et/ou des nacres.

**31.** Composition selon l'une quelconque des revendications précédentes, **caractérisée en ce qu'**elle comprend au moins un colorant hydrosoluble ou liposoluble.

**32.** Composition selon l'une quelconque des revendications précédentes, **caractérisée en ce qu'**elle comprend au moins une matière colorante.

**33.** Composition selon l'une quelconque des revendications précédentes, **caractérisée en ce qu'**elle comprend au moins un additif choisis parmi les polymères, notamment les polymères filmogènes, les polymères fixants ; les tensioactifs ; les agents conditionneurs des cheveux ; les matières colorantes ; les agents nacrants ; les opacifiants ; les solvants organiques ; les parfums ; les épaississants ; les gélifiants ; les cires ; les produits pâteux ; les colorants capillaires ; les résines de silicones ; les gommes de silicone, les conservateurs ; les antioxydants, les actifs cosmétiques ; les filtres solaires ; les agents de stabilisation du pH ; les vitamines ; les hydratants ; les agents antitranspirants ; les agents déodorants ; les composés auto-bronzants et leurs mélanges.

**34.** Utilisation d'au moins un ester d'acide carbonique répondant à la formule (I) suivante :

$$R_1\text{-}O\text{-}CO\text{-}O\text{-}R_2 \qquad (I)$$

dans laquelle R1 et R2 sont identiques ou différents et représentent un radical hydrocarboné linéaire ou ramifié, avec le proviso que

- lorsque R1 et R2 sont linéaires, ils contiennent chacun de 1 à 6 atomes de carbone avec $6 \leq R_1 + R_2 \leq 7$ ; et
- lorsque R1 et/ou R2 sont ramifiés, ils contiennent chacun de 1 à 8 atomes de carbone avec $6 \leq R_1 + R_2 \leq 9$,

en tant que solvant volatil pour la préparation d'une composition cosmétique.

**35.** Utilisation selon la revendication 34, **caractérisée en ce que** l'ester d'acide carbonique est tel que défini selon l'une quelconque des revendications 1 à 14.

**36.** Procédé cosmétique de maquillage et/ou de soin de la peau des lèvres et/ou des fibres kératiniques comprenant au moins l'étape d'application sur la peau, les lèvres et/ou les fibres kératiniques d'une composition telle que définie selon l'une quelconque des revendications 1 à 33.

**Office européen des brevets**

## RAPPORT DE RECHERCHE EUROPEENNE

Numéro de la demande

EP 07 12 1283

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | JP 08 333599 A (OLYMPUS OPTICAL CO) 17 décembre 1996 (1996-12-17) * abrégé * ----- | 1-34 | INV. A61K8/37 A61Q1/06 A61Q19/00 |
| X | US 6 506 243 B1 (YEZRIELEV ALBERT ILYA [US] ET AL) 14 janvier 2003 (2003-01-14) * exemples 3c-3f * ----- | 1-34 | |
| X | US 5 814 163 A (WOJCIK GERALD [US]) 29 septembre 1998 (1998-09-29) * revendications 1,10 * ----- | 1-34 | |
| X | US 5 895 644 A (ALBANESE JOSEPH [US] ET AL) 20 avril 1999 (1999-04-20) * colonne 9, ligne 29-31; revendications * ----- | 1-34 | |
| X | US 4 447 365 A (BODEN RICHARD M [US] ET AL) 8 mai 1984 (1984-05-08) * revendications; exemples V-XVI * ----- | 1-34 | |
| X | EP 1 512 392 A (COGNIS DEUTSCHLAND GMBH [DE]) 9 mars 2005 (2005-03-09) * revendications * ----- | 1-34 | DOMAINES TECHNIQUES RECHERCHES (IPC) A61K A61Q |
| X | WO 02/05759 A1 (COGNIS DEUTSCHLAND GMBH [DE]) 24 janvier 2002 (2002-01-24) * revendications * ----- | 1-34 | |
| X | DE 41 19 890 A1 (HENKEL KGAA [DE]) 24 décembre 1992 (1992-12-24) * revendications * ----- | 1-34 | |

Le présent rapport a été établi pour toutes les revendications

| Lieu de la recherche | Date d'achèvement de la recherche | Examinateur |
|---|---|---|
| Munich | 28 février 2008 | Miller, Bernhard |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 07 12 1283

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

28-02-2008

| Document brevet cité au rapport de recherche | | Date de publication | Membre(s) de la famille de brevet(s) | | Date de publication |
|---|---|---|---|---|---|
| JP 8333599 | A | 17-12-1996 | AUCUN | | |
| US 6506243 | B1 | 14-01-2003 | AUCUN | | |
| US 5814163 | A | 29-09-1998 | AUCUN | | |
| US 5895644 | A | 20-04-1999 | AT | 214910 T | 15-04-2002 |
| | | | AU | 745332 B2 | 21-03-2002 |
| | | | AU | 1528199 A | 15-06-1999 |
| | | | BG | 104536 A | 30-04-2001 |
| | | | BR | 9814231 A | 03-10-2000 |
| | | | CA | 2310787 A1 | 03-06-1999 |
| | | | CN | 1283984 A | 14-02-2001 |
| | | | CZ | 20001881 A3 | 11-10-2000 |
| | | | DE | 69804493 D1 | 02-05-2002 |
| | | | DE | 69804493 T2 | 14-11-2002 |
| | | | DK | 1032360 T3 | 22-07-2002 |
| | | | EP | 1032360 A1 | 06-09-2000 |
| | | | ES | 2175825 T3 | 16-11-2002 |
| | | | HU | 0100446 A2 | 30-07-2001 |
| | | | JP | 2001523700 T | 27-11-2001 |
| | | | NO | 20002579 A | 19-05-2000 |
| | | | NZ | 504580 A | 27-09-2002 |
| | | | PL | 340580 A1 | 12-02-2001 |
| | | | PT | 1032360 T | 30-09-2002 |
| | | | RU | 2213556 C2 | 10-10-2003 |
| | | | WO | 9926598 A1 | 03-06-1999 |
| US 4447365 | A | 08-05-1984 | AUCUN | | |
| EP 1512392 | A | 09-03-2005 | DE | 10341025 A1 | 31-03-2005 |
| | | | JP | 2005075833 A | 24-03-2005 |
| | | | US | 2005079986 A1 | 14-04-2005 |
| WO 0205759 | A1 | 24-01-2002 | AU | 2002227526 A1 | 30-01-2002 |
| | | | BR | 0112537 A | 01-07-2003 |
| | | | CA | 2418336 A1 | 17-01-2003 |
| | | | CN | 1443057 A | 17-09-2003 |
| | | | DE | 10035071 A1 | 31-01-2002 |
| | | | EP | 1301160 A1 | 16-04-2003 |
| | | | JP | 2004503571 T | 05-02-2004 |
| | | | US | 2003180374 A1 | 25-09-2003 |
| DE 4119890 | A1 | 24-12-1992 | AU | 2017992 A | 12-01-1993 |
| | | | WO | 9222282 A1 | 23-12-1992 |
| | | | MX | 9202902 A1 | 01-12-1992 |

EPO FORM P0460

Pour tout renseignement concernant cette annexe : voir Journal Officiel de l'Office européen des brevets, No.12/82

**EP 1 925 292 A1**

## RÉFÉRENCES CITÉES DANS LA DESCRIPTION

**Documents brevets cités dans la description**

- WO 06013413 A
- EP 542669 A
- EP 787730 A
- EP 787731 A
- WO 9608537 A